# EUROPEAN PATENT SPECIFICATION

(11) **EP 3 721 886 B1**
(45) Date of publication and mention of the grant of the patent: **10.09.2025**
(21) Application number: 18885300.6
(22) Date of filing: 07.12.2018
(51) Int. Cl.: A61K 33/00, A61P 7/00, A61P 31/00, A61P 35/00

(54) **THERAPEUTIC METHOD**
THERAPEUTISCHES VERFAHREN
MÉTHODE THÉRAPEUTIQUE

(30) Priority: 08.12.2017 JP 2017236430
(43) Date of publication of application: 14.10.2020
(73) Proprietor: Ohdaira, Takeshi, Kumagaya-shi, Saitama 360-0014 (JP)
(72) Inventor: Ohdaira, Takeshi, Kumagaya-shi, Saitama 360-0014 (JP)
(74) Representative: Pons IP
(86) International application number: PCT/JP2018/045184
(87) International publication number: WO 2019/112061

(56) References cited:
- WO-A1-2004/021907
- WO-A1-2008/072370
- WO-A1-2008/072371
- WO-A1-2013/187407
- WO-A1-2017/195852
- WO-A1-2017/199827
- JP-A- 2005 245 817
- JP-A- 2009 084 258
- JP-A- 2011 105 642
- US-A1- 2007 189 972
- US-A1- 2015 174 066
- TSUGE, HIDEKI: "Fundamentals of microbubbles and nanobubbles", BULLETIN OF THE SOCIETY OF SEA WATER SCIENCE
- CHOWDHURY, SAYAN MULLICK ET AL.: "Ultrasound-guided drug delivery in cancer", ULTRASONOGRAPHY, vol. 36, no. 3, July 2017 (2017-07-01), pages 171 - 184, XP055614505

## Description

### Technical Field

The present invention relates to a microbubble dispersion liquid for use in therapy.

### Background Art

A nanobubble dispersion liquid contains fine bubbles with a diameter of less than 1 µm. Because it exhibits the effect to promote the biological activity of living organisms, the nanobubble dispersion liquid has attracted attention from various industrial fields. As prior art, Patent Document 1 (JP-A-2009-131768) and Patent Document 2 (Japanese Patent No. 4144669) describe methods for producing a nanobubble dispersion liquid.

WO 2017/199827 A1 discloses an aqueous solution administrable to an organism containing ozone nanobubbles of 30 nm or less, and indicates that said aqueous solution is used in the treatment of microbial infection or viral infection of tissues in an organism, and the prevention of the occurrence, or control of the occurrence, of microbes or viruses, and that said aqueous solution is administered to an organism to suppress or prevent proliferation and enlargement of cancer cells.

However, such prior-art documents fail to disclose applying the nanobubble dispersion liquid to the medical field. Microbubbles, which have a larger particle size than nanobubbles, will exhibit the same effect as nanobubbles, but the prior-art documents also fail to disclose applying a microbubble dispersion liquid to the medical field.

### Prior-art Document

### Patent Document

Patent Document 1: JP-A-2009-131768
Patent Document 2: Japanese Patent No.: 4144669
Patent Document 3: WO 2017/199827 A1

### Summary of the Invention

### Problems to be Solved by the Invention

The present invention intends to solve the problems involved in the prior art discussed above, and to provide a means so that it can be used effectively in the medical field.

### Means for Solving Problems

The references to the methods of treatment in this description are to be interpreted as references to microbubble dispersion liquids of the present invention for use in those methods.

A first therapeutic method is described, but not claimed, including bringing a nanobubble dispersion liquid containing nanobubbles into contact with an affected area, wherein the nanobubbles are positively or negatively charged, and have an average particle size of 10 nm to 500 nm.

In the first therapeutic method, bringing the nanobubble dispersion liquid into contact with the affected area is preferable to improve an antibacterial effect against pathogenic microorganisms in the affected area.

In the first therapeutic method, bringing the nanobubble dispersion liquid into contact with the affected area is preferable to crush tumor cells in the affected area.

A second therapeutic method of the present invention includes bringing a microbubble dispersion liquid containing microbubbles into contact with an affected area, wherein the microbubbles are positively or negatively charged, and have an average particle size of 1 µm to 900 µm.

In the second therapeutic method, not claimed, bringing the microbubble dispersion liquid into contact with the affected area is preferable to crush and wash bloody or non-bloody infectious microbubbles, or bloody or non-bloody infectious exudate in the affected area by a ballooning effect of the microbubbles.

In the second therapeutic method, not claimed, bringing the microbubble dispersion liquid into contact with the affected area is preferable to crush and remove a blood clot in the affected area by the ballooning effect of the microbubbles.

In the second therapeutic method, bringing the microbubble dispersion liquid into contact with the affected area is to exfoliate and remove tumor cells in the affected area by the ballooning effect of the microbubbles.

A third therapeutic method of the present invention includes bringing a mixed liquid obtained by mixing a nanobubble dispersion liquid containing nanobubbles and a microbubble dispersion liquid containing microbubbles into contact with an affected area, wherein the nanobubbles are positively or negatively charged, and have an average particle size of 10 nm to 500 nm, and the microbubbles are positively or negatively charged, and have an average particle size of 1 µm to 900 µm.

### Advantageous Effects of the Invention

Thus, according to the present invention, a nanobubble dispersion liquid containing nanobubbles (not claimed) and/or a microbubble dispersion liquid containing microbubbles can be used effectively in the medical field.

### Brief Description of the Drawings

Fig. 1 is a schematic diagram that illustrates the action of microbubbles on viscous excrement or infectious exudate.
Fig. 2 is a diagram that compares time required for each cancer cell line to be exfoliated from a serosa in an electrostatically-charged microbubble dispersion liquid and in a control liquid.

### Description of Embodiments

The parts of the description relating to nanobubble dispersion liquid are not according to the claims. The invention relates to a microbubble dispersion liquid for use in therapy for exfoliating and removing tumor cells in the affected area by a ballooning effect of the said microbubbles through bringing the microbubble dispersion liquid into contact with the affected area, wherein the microbubble dispersion liquid contains, positively or negatively microbubbles,said microbubbles having an average particle size of 1 µm to 900 µm; and being dispersed on an order of 10⁵ to 10¹⁰ in 1 cc of the microbubble dispersion liquid.

This section will describe embodiments of the present invention with reference to the accompanying drawings.

Treatment of an affected area by bringing a nanobubble dispersion liquid, a microbubble dispersion liquid, or a mixed liquid of these dispersion liquids into contact with the affected area is disclosed.

Dispersing nanobubbles in a liquid forms the nanobubble dispersion liquid. The nano-order fine bubbles used for the present invention have an average particle size of 10 nm to 500 nm. Bubbles having an average particle size of less than 10 nm are not preferable because the acting force exerted on tissue by bubbles having such a size is too small to accomplish medical objectives. Also, bubbles having an average particle size of more than 500 nm are not preferable because the degree of dispersibility in a liquid decreases for bubbles having such a size. This causes the acting force exerted on the tissue to be too small to accomplish medical objectives.

Further, the nanobubbles are dispersed in a liquid in a positively or negatively charged state. Positively or negatively charged nanobubbles repel each other, resulting in improved bubble dispersibility in the liquid. This allows the nanobubbles to contact thoroughly with the affected area. Contact of nanobubbles with the affected area can be achieved by applying nanobubble dispersion liquid to the affected area, applying gauze impregnated with the liquid onto the affected area, or immersing the affected area in the liquid.

It is preferable that the number of nanobubbles contained in 1 cc of the liquid is on the order of 10⁵ to 10¹⁰. This content level provides a good acting force of nanobubbles exerted on concerned tissue. Further, it is preferable that nanobubbles have a zeta potential of 10 mV to 200 mV. If nanobubbles have zeta potential deviating from this range, the degree of bubble dispersibility decreases, which is not preferable.

Ozone water or sterilized water can be selected for the liquid in which nanobubbles are dispersed. Ozone can be used as nanobubbles in ozone water.

Bringing such a nanobubble dispersion liquid into contact with the tissue of an affected area is used to treat this area. Bringing nanobubble dispersion liquid into contact with tissue of the affected area can improve antibacterial effect in such a way that the liquid sterilizes pathogenic microorganisms.

This antibacterial effect is achieved as charged nanobubbles bond electrostatically to and puncture the cell membrane of pathogenic microorganisms to destroy it, whereby intracellular structures including the nucleus escape from the cells, which will die. Nanobubble dispersion liquid containing ozone also destroys the cell membrane. Further, charged nanobubbles come into contact and enter cells at the affected area by endocytosis or phagocytosis and are transported to intracellular lysosomes to destroy them, so that protoplasm of digestive enzymes in the lysosomes is lost, and the cells are destroyed from inside the cells.

Bringing the nanobubble dispersion liquid into contact with tumor cells such as cancer cells can crush and kill the tumor cells.

As is the case for pathogenic microorganisms, charged nanobubbles coming into contact with tumor cells such as cancer cells electrostatically bond to the tumor cells and puncture the cell membrane to destroy it, whereby intracellular structures including the cell nucleus escape from the cells, which will die. Nanobubble dispersion liquid containing ozone also destroys the cell membrane. Nanobubble dispersion liquid exhibits antitumor effects because of the aforementioned mechanism.

Also, charged nanobubbles coming into contact with tumor cells enter the cells by endocytosis or phagocytosis and are transported to intracellular lysosomes of the tumor cells to destroy them, so that protoplasm of digestive enzymes in the lysosomes is lost, and tumor cells are destroyed from inside the cells. Nanobubble dispersion liquid also exhibits antitumor effects because of this mechanism.

A microbubble dispersion liquid can be used in addition to the nanobubble dispersion liquid in the present invention. Microbubble dispersion liquid is formed by dispersing micro-order fine bubbles having an average particle size of 1 µm to 900 µm in a liquid. Bubbles having an average particle size of less than 1 µm are not preferable because the acting force exerted on the tissue by bubbles having such a size becomes too small to accomplish medical objectives of the present invention. Also, bubbles having an average particle size of more than 900 µm are not preferable because the degree of dispersibility in a liquid decreases for bubbles having such a size. This causes the acting force exerted on the tissue to become too small to accomplish medical objectives of the present invention.

Microbubbles are dispersed in a liquid in a positively or negatively charged state. Positively or negatively charged microbubbles repel each other, resulting in improved bubble dispersibility in the liquid. This allows the microbubbles to contact thoroughly with the tissue.

In addition, it is preferable that the number of microbubbles contained in 1 cc of the liquid is on the order of 10⁵ to 10¹⁰ as with the nanobubbles. This content level provides a good acting force of microbubbles exerted on concerned tissue. Further, it is preferable that microbubbles have a zeta potential of 10 mV to 200 mV. If nanobubbles have zeta potential deviating from this range, the degree of bubble dispersibility decreases, which is not preferable.

If the affected area is bloody or non-bloody infectious viscous excrement, or bloody or non-bloody infectious exudate (not according to the claims), microbubble dispersion liquid can be brought into contact with the bloody or non-bloody infectious viscous excrement, or bloody or non-bloody infectious exudate of the affected area.

The microbubble dispersion liquid exhibits a "ballooning effect." In this phenomenon microbubbles expand to a size of several mm within a few seconds to a few tens of seconds. The microbubbles crush the excrement or the exudate to the range of several µm to several hundred µm by the ballooning effect if the microbubble dispersion liquid comes into contact with the bloody or non-bloody infectious viscous excrement or exudate. This ballooning effect can significantly improve efficiency to wash the affected area.

Specifically, charged microbubbles have an attractive Coulomb force. As a result, the microbubble dispersion liquid balances entropy-increasing force of gas molecules in bubbles with the attractive Coulomb force, which controls the tendency for bubble-diameter to increase. Thus, viscous excrement and exudate are crushed by the ballooning effect caused by the microbubbles. Accordingly, microbubble dispersion liquid can significantly improve cleaning efficiency of the affected area.

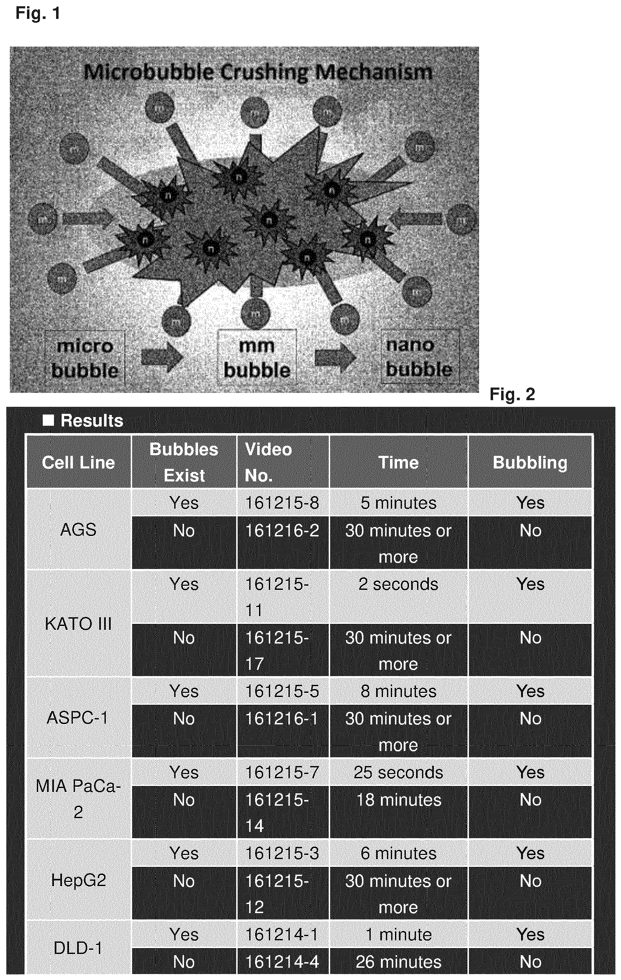

Fig. 1 is a schematic diagram that illustrates the action of microbubbles on viscous excrement or infectious exudate (not according to the invention). Microbubbles enter inside viscous secretions such as sputum by taking advantage of the electrostatic properties and small size of the microbubbles, and crush viscous secretions from the inside by the microbubbles ballooning effect. At this time, microbubbles crush the viscous secretions leaving only fibers, allowing the microbubbles to wash the viscous secretions promptly and completely. As a result, debridement (exclusion and washing of abnormal granulations and necrotic tissues) is completed in a short time, making it possible to immediately and completely remove local contaminants that delay wound healing at a macroscopic level.

If the affected area is a blood clot (not according to the claims), microbubbles can crush the blood clot so that the clot is removed by the microbubbles ballooning effect when bringing microbubble dispersion liquid into contact with the blood clot. This mechanism is the same as described previously with reference to Fig. 1.

Microbubbles enter inside the blood clot by taking advantage of the electrostatic properties and small size of the microbubbles, break up the blood clot from the inside by the microbubbles ballooning effect, and crush the blood clot leaving only fibers. As a result, the microbubbles can wash the blood clot promptly and completely. Thus, during an operation this mechanism secures a field of view by washing the blood clot.

Bleeding from the spleen typically results in giant hematomas and bloody ascites, which will fill the abdominal cavity. Conventionally, a jelly-like clot remains no matter how many minutes are spent for washing the abdominal cavity with saline, which will clog the washing nozzle. With the microbubble dispersion liquid all the blood clot will be finally crushed, even after the abdominal cavity has filled with giant hematomas and bloody ascites due to bleeding from the spleen, and even though clouding by misty saline occurs for five seconds while the ballooning effect is exerted after the microbubble dispersion liquid has been applied. As a result, body cavity cleaning can be performed while a clear field of view is secured, making it possible to return quickly to a surgical operation and continue the operation.

If the affected area consists of tumor cells including cancer cells, microbubbles exfoliate and remove the tumor cells by the microbubbles ballooning effect when bringing the microbubble dispersion liquid into contact with the tumor cells. This mechanism will be explained in the following, using cancer cells as an example.

Cancer cells adhere by cell adhesion factors to the serosa including the peritoneum, pleura, and pericardium, which will establish cancer serosa-disseminated metastasis in the serosa. If the microbubble dispersion liquid is brought into contact with the cancer cells, the electrostatically-charged microbubbles can enter between the cancer cells and the serosa due to the small size of the microbubbles to electrostatically bond with the cells and the serosa, so that microbubbles exist between the cancer cells and the serosa. Then, the microbubbles between the cancer cells and the serosa lift up the cancer cells in the same manner as a jack for vehicles. As a result, cancer cells that adhered to the serosa by cell adhesion factor are exfoliated from the serosa, and this prevents or removes the cancer serosa-disseminated metastasis.

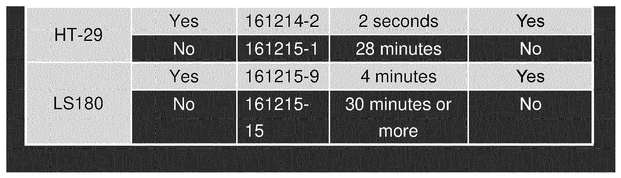

Fig. 2 is a diagram that compares the time required for each cancer cell line to be exfoliated from a serosa in an electrostatically-charged microbubble dispersion liquid and in a control liquid. The diagram shows the superiority of electrostatically-charged microbubble dispersion liquid in terms of exfoliation/washing properties for all the cancer cell lines tested. Cancer cell lines shown in Fig. 2 are the subject of the following respective surgeries: "AGS" is gastric adenocarcinoma, "KATO III" is gastric cancer, "ASPC-1" is metastatic pancreatic cancer, "MIAPaCa-2" is pancreatic cancer, "HepG2" is liver cancer, and "DID-1", "HT-29", "LS180" are respective colon adenocarcinoma.

In the present invention, a mixed liquid obtained by mixing nanobubble dispersion liquid and microbubble dispersion liquid can be used for treatment by bringing the mixed liquid into contact with the affected area. If the mixed liquid is used, effects can be exhibited of both the nanobubble dispersion liquid and the microbubble dispersion liquid. That is, (1) the nanobubble dispersion liquid can improve the antibacterial effect as well as destroy tumor cells, and (2) the microbubble dispersion liquid can (i) crush and wash bloody or non-bloody infectious viscous excrement or exudate, (ii) crush and remove blood clots, and (iii) exfoliate and remove tumor cells.

## Claims

1. A microbubble dispersion liquid for use in therapy for exfoliating and removing tumor cells in the affected area by a ballooning effect of the said microbubbles through bringing the microbubble dispersion liquid into contact with the affected area,
wherein the microbubble dispersion liquid contains,
positively or negatively microbubbles,
said microbubbles having an average particle size of 1 µm to 900 µm; and
being dispersed on an order of 10⁵ to 10¹⁰ in 1 cc of the microbubble dispersion liquid.

2. A microbubble dispersion liquid according to Claim 1, wherein the tumor cells are at least one of: "AGS" gastric adenocarcinoma, "KATO III" gastric cancer, "ASPC-1" metastatic pancreatic cancer, "MIAPaCa-2" pancreatic cancer, "HepG2" liver cancer, and "DID-1", "HT-29", "LS180" types of colon adenocarcinoma cells.

## Patentansprüche

1. Mikrobläschen-Dispersionsflüssigkeit zur Verwendung bei der Therapie zum Exfolieren und Entfernen von Tumorzellen in dem betroffenen Bereich durch eine Ballonwirkung der Mikrobläschen, indem die Mikrobläschen-Dispersionsflüssigkeit mit dem betroffenen Bereich in Kontakt gebracht wird, wobei die Mikrobläschen-Dispersionsflüssigkeit Folgendes enthält:
positiv oder negativ ## Mikrobläschen,
wobei die Mikrobläschen eine durchschnittliche Teilchengröße von 1 µm bis 900 µm aufweisen; und
in einer Größenordnung von 10⁵ bis 10¹⁰ in 1 cm³ der Mikrobläschen-Dispersionsflüssigkeit dispergiert sind.

2. Mikrobläschen-Dispersionsflüssigkeit nach Anspruch 1, wobei die Tumorzellen mindestens eines von folgenden sind: Zellen eines Adenokarzinoms des Magens (AGS), eines Magenkarzinoms (KATO III), eines metastasierten Pankreaskarzinoms (ASPC-1), eines Pankreaskarzinoms (MIAPaCa-2), eines Leberkarzinoms (HepG2) und von Arten eines Adenokarzinoms des Dickdarms (DID-1, HT-29, LS180).

## Revendications

1. Liquide de dispersion de microbulles destiné à être utilisé en thérapie pour l'exfoliation et l'élimination de cellules tumorales dans la zone affectée par un effet de gonflement desdites microbulles en mettant le liquide de dispersion de microbulles en contact avec la zone affectée,
dans lequel le liquide de dispersion de microbulles contient,
des microbulles positives ou négatives,
lesdites microbulles ayant une taille moyenne de 1 µm à 900 µm ; et
étant dispersées dans un ordre de 10⁵ à 10¹⁰ dans 1 cc du liquide de dispersion de microbulles.

2. Liquide de dispersion de microbulles selon la revendication 1, dans lequel les cellules tumorales sont au moins l'un parmi : l'adénocarcinome gastrique « AGS », le cancer gastrique « KATO III », le cancer du pancréas métastatique « ASPC-1 », le cancer du pancréas « MIAPaCa-2 », le cancer du foie « HepG2 », et les types de cellules d'adénocarcinome colorectal « DID-1 », « HT-29 », « LS180 ».
